# EUROPEAN PATENT APPLICATION

(11) **EP 4 563 143 A1**
(43) Date of publication of application: **04.06.2025**
(21) Application number: 24215997.8
(22) Date of filing: 28.11.2024
(51) Int. Cl.: A61K 9/20, A61K 9/24, A61K 31/155, A61K 31/4985, A61P 3/10

(54) **A BILAYER TABLET FORMULATION OF METFORMIN AND SITAGLIPTIN COMPRISING ANTIOXIDANT**

(30) Priority: 30.11.2023 TR 202316130
(71) Applicant: Sanovel Ilac Sanayi ve Ticaret A.S., Sisli - Istanbul (TR)
(72) Inventor: SUNEL, Fatih, Istanbul (TR); PEHLIVAN AKALIN, Nur, Istanbul (TR); TUNC, GULDENIZ, Istanbul (TR)
(74) Representative: Genç Ilhan, Oznur

(57) **Abstract**

The present invention relates to a bilayer tablet comprising extended-release formulation having metformin or a pharmaceutically acceptable salt thereof and at least one cellulose derivates as binder and immediate release formulation having sitagliptin or a pharmaceutically acceptable salt thereof and at least one antioxidant. The present invention also relates to a simple, rapid, cost effective, time-saving and industrially convenient process of preparing the bilayer tablet.

## Description

### Field of Invention

The present invention relates to a bilayer tablet comprising extended-release formulation having metformin or a pharmaceutically acceptable salt thereof and at least one cellulose derivates as binder and immediate release formulation having sitagliptin or a pharmaceutically acceptable salt thereof and at least one antioxidant. The present invention also relates to a simple, rapid, cost effective, time-saving and industrially convenient process of preparing the bilayer tablet.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether, and chloroform. Oral doses of metformin are generally recommended in the range of 500 to 2500 mg a day and a single dose may vary from 500 to 850 mg. It is used singly or in combination with sulfonylureas, alpha-glucosidase inhibitors, or insulin.

The chemical name of metformin hydrochloride is 1,1-dimethyl biguanide hydrochloride, has the following chemical structure of Formula I.

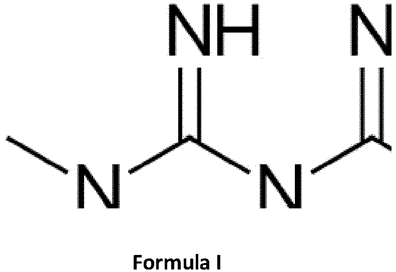

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine and its chemical structure is shown in Formula II.

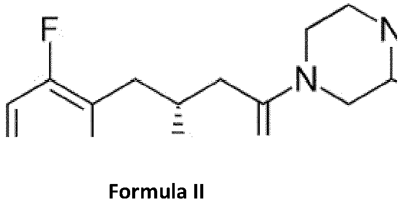

Sitagliptin is disclosed in the U.S. Patent No. 6699871. A crystal phosphate monohydrate form of sitagliptin is disclosed in the patent WO 2005003135.

DPP-4 inhibitors, especially sitagliptin have a novel mechanism of action and many studies showing their efficacy and safety in medication are available.

Extended-release formulations of metformin have advantages over immediate release in terms of affording a more uniform maintenance of blood plasma active drug concentrations and providing better patient compliance by reducing the frequency of administration required.

Extended-release formulations comprising metformin are disclosed in several other U.S. 20 Patent No. 6,635,280 and U.S. Patent No. 6,866,866 and U.S. Patent No. 6,660,300.

Sitagliptin-metformin extended-release tablets are composed of sitagliptin phosphate monohydrate and metformin hydrochloride, and are compound preparations used for the treatment of type 2 diabetes.

A tablet formulation for sitagliptin and extended-release metformin combination is commercially available under the trade name Janumet XR^{®}.

The patent US8758815B2 discloses a pharmaceutical composition, preferably a pharmaceutical dosage form, comprising at least two separate compartments, wherein one compartment contains a composition comprising metformin or a pharmaceutically acceptable salt thereof and wherein another compartment contains a composition comprising sitagliptin.

The patent CN114042051A respectively granulating the metformin hydrochloride and the sitagliptin phosphate and then pressing into bilayer tablet according to the needed proportion. The method developed for stability and the formulation created are explained.

There is also still a need for a bilayer tablet form comprising metformin and sitagliptin having desired dissolution profile and stability.

In the prior art, different formulation studies are still being carried out for stability and dissolution profile. Antioxidant has been used in the prior art in the metformin layer for stability issues. In the present invention, the combination does not comprise antioxidant in the metformin layer. We found that when antioxidants were used in the metformin layer, it negatively affected the flowability of metformin. The use of antioxidants in the sitagliptin layer provided the desired stability and we found that it did not cause any problems by reacting with the formulation containing sitagliptin.

Furthermore, we overcame some problems (flowability, compressibility and content uniformity) caused by the use of high amounts of metformin by using at least two cellulose derivative binders. Thus, we easily achieved the desired dissolution profile and stability.

### Detailed description of the Invention

The main object of the present invention is to provide a bilayer tablet comprising metformin and sitagliptin having the desired dissolution profile and high stability.

Another object of the present invention is to provide a bilayer tablet comprising metformin and sitagliptin that does not have problems such as content uniformity, flowability and compressibility.

Another object of the present invention is to provide improved processes for preparing a bilayer tablet comprising metformin and sitagliptin.

As used herein, the term "metformin" includes metformin and pharmaceutically acceptable salts thereof. The pharmaceutically acceptable salts include hydrochloride, hydrobromide, fumarate, malonate, malate, succinate, lactate, glycolate, maleate, citrate, and aspartate. The pharmaceutically acceptable salts of metformin may be present in hydrous or anhydrous forms. They may also be present in crystalline or amorphous forms. In this invention, preferably metformin hydrochloride is used.

As used herein, the term "sitagliptin" includes sitagliptin and pharmaceutically acceptable salts thereof. The pharmaceutically acceptable salts include hydrochloride, hydrobromide, phosphate, sulphate, mesylate, besylate, fumarate, malonate, malate, succinate, lactate, glycolate, maleate, citrate, and aspartate. The pharmaceutically acceptable salts of sitagliptin may be present in hydrous or anhydrous forms. The hydrate forms may be monohydrate or dihydrate forms. The pharmaceutically acceptable salts of sitagliptin may also be present in crystalline or amorphous forms. In this invention, preferably sitagliptin phosphate monohydrate is used.

In this invention, we aimed to eliminate the negative effects such as flowability, compressibility and content uniformity that may be caused by high amounts of metformin by using at least two cellulose-derived binders in the metformin layer. Also, we ensured stability in the sitagliptin layer by using at least one antioxidant. Thus, we achieved the desired stability and dissolution profile.

According to this embodiment of the present invention, a bilayer tablet comprises;
- First layer comprising extended-release formulation comprising metformin or a pharmaceutically acceptable salt thereof and at least one cellulose derivates as binder,
- Second layer comprising immediate release formulation comprising sitagliptin or a pharmaceutically acceptable salt thereof and at least one antioxidant.

According to an embodiment of the present invention, at least one cellulose derivate as binder is selected from the group comprising hydroxypropylcellulose, methyl cellulose, ethyl cellulose, hydroxypropyl methylcellulose, carboxymethylcellulose sodium or mixtures thereof.

According to this embodiment of the invention, the first layer comprises two binders. The binders are carboxymethylcellulose sodium and hydroxypropyl methylcellulose. These binders helped in the extended release of the separator, providing the desired dissolution profile. Furthermore, using these excipients provide flowability, compressibility and content uniformity.

According to this embodiment of the invention, the amount of the binders in the first layer is between 1.0% and 7.0% by weight. Preferably, the amount of the binders in the first layer is between 2.0% and 5.0% by weight in the total formulation.

According to this embodiment of the invention, antioxidant is used only at the second layer. Antioxidants are selected from the radical group, vitamins and salts, thiols and polyphenols.

According to this embodiment of the invention, suitable antioxidants are selected from the radical group which comprises meglumine, arginine, butylated hydroxy toluene, butylated hydroxy anisole, sodium metabisulfite and sodium bisulfite.

According to this embodiment of the invention, suitable antioxidants are selected from the vitamins and salts which are alpha tocopherol, ascorbic acid, sodium ascorbate, ascorbyl palmitate and erythorbic acid.

According to this embodiment of the invention, suitable antioxidants are selected from the thiols which are thioglycerols, thiogallic acid, cysteine, glutathione, cysteamine, dihydrolipoic acid, lipoic acid and thioredoxin.

According to this embodiment of the invention, suitable antioxidants are selected from the polyphenols which are propyl gallate, ethyl gallate, methyl gallate and lauryl gallate.

Suitable antioxidants are selected from the group comprising propyl gallate, meglumine, arginine, butylated hydroxy toluene, butylated hydroxy anisole, sodium metabisulfite, sodium bisulfite, alpha tocopherol, ascorbic acid, sodium ascorbate, ascorbyl palmitate, erythorbic acid, thioglycerols, thiogallic acid, cysteine, glutathione, cysteamine, dihydrolipoic acid, lipoic acid, thioredoxin, ethyl gallate, methyl gallate, lauryl gallate or mixtures thereof.

According to this embodiment of the invention, antioxidant is propyl gallate or meglumine or arginine or mixture thereof.

According to this embodiment of the invention, antioxidant is propyl gallate.

According to this embodiment of the invention, the amount of antioxidants is 0.01% to 5.0% by weight in the total formulation. Preferably, the amount of antioxidants is between 0.05% and 2.0% by weight in the total formulation.

According to this embodiment of the invention, the amount of metformin is 25.0% to 80.0% by weight in the total formulation. Preferably, the amount of metformin is between 35.0% and 65.0% by weight in the total formulation.

According to this embodiment of the invention, the amount of sitagliptin is 1.0% to 15.0% by weight in the total formulation. Preferably, the amount of sitagliptin is between 2.0% and 10.0% by weight in the total formulation.

According to an embodiment of the present invention, the bilayer tablet further comprises at least one pharmaceutically acceptable excipient which is selected from the group comprising binders, matrix agents, fillers, lubricants, antioxidants, coating agents or mixtures thereof.

According to an embodiment of the present invention, at least one binder is used at second layer. Suitable binders are selected from the group comprising polyvinylpyrrolidone (povidone), lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

According to this embodiment of the invention, the second layer comprises a binder which is polyvinylpyrrolidone.

According to this embodiment of the invention, the amount of the binder in the second layer is between 0.05% and 5.0% by weight.

Suitable matrix agents are selected from the group comprising hydroxypropylmethyl cellulose, ethyl cellulose, polymethylmethacrylate derivatives poloxamer polyvinyl alcohol, xanthan gum, sodium alginate, polymethacrylates, polyacrylamide, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, diethyl aminoethyl methacrylate or mixtures thereof.

According to an embodiment of the present invention, the first layer comprises at least one matrix agent which is hydroxypropyl methyl cellulose (K100 MCR/ K100M/ CN10T). Preferably, the matrix agent is hydroxypropyl methyl cellulose K100 MCR.

According to an embodiment of the present invention, the amount of matrix agents is 10.0% to 35.0% by weight in the total formulation. Preferably, the amount of matrix agent is between 15.0% and 30.0% by weight in the total formulation.

Suitable fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to this embodiment of the present invention, filler is present at first layer or second layer or mixtures thereof.

According to this embodiment of the present invention, the filler is microcrystalline cellulose. Microcrystalline cellulose is present at first layer or second layer or mixtures thereof. Preferably, Microcrystalline cellulose is present at both layers.

According to this embodiment of the present invention, the amount of fillers are between 5.0% and 25.0% by weight in the total formulation. Preferably, the amount of fillers are between 7.0% and 15.0% by weight in the total formulation.

According to this embodiment of the invention, the weight of the fillers in the first layer is between 5.0% and 15.0% by weight in the first layer.

According to this embodiment of the invention, the amount of the fillers in the second layer is between 60.0% and 75.0% by weight in the second layer.

Suitable lubricants are selected from the group comprising sodium stearyl fumarate, talc, magnesium stearate, calcium stearate, zinc stearate, magnesium lauryl sulfate, sodium oleate, polyethylene glycol, sodium lauryl sulphate or mixtures thereof.

According to an embodiment of the present invention, the lubricant is present at both layers.

According to an embodiment of the present invention, the lubricant is sodium stearyl fumarate. Sodium stearyl fumarate is used in both layers.

According to this embodiment of the present invention, the amount of lubricants are between 0.04% and 4.0% by weight in the total formulation. Preferably, the amount of lubricants are between 0.1% and 2.0% by weight in the total formulation.

According to this embodiment of the invention, the amount of coating agent is 0. 1% to 5.0% by weight in the total formulation. Preferably, the amount of coating agent is between 1.0% and 3% by weight in the total formulation.

The bilayer tablet comprising metformin or salts thereof and sitagliptin or salts thereof of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

In the present invention, wet granulation method is used for the formulation comprising metformin for elimination of compressibility problems related to metformin and dry granulation is used for the formulation comprising sitagliptin.

### Example 1:

| **Ingredients** | **%by weight** | **%by weight** |
|---|---|---|
| Metformin HCL | 60 | 40-80 |
| Sodium Carboxymethyl Cellulose (7 HF) | 3 | 1-7 |
| Hydroxypropyl methylcellulose (K100 LV) | 1.2 | 0.05-4 |
| Hydroxypropyl methylcellulose K100 MCR/ K100M/ CN10T | 16.4 | 10-35 |
| Sodium stearyl fumarate | 0.4 | 0.02-2 |
| **FIRST LAYER TABLET WEIGHT** | **81** | **75-85** |
| Sitagliptin phosphate monohydrate (eq to 100mg Sitagliptin) | 7.7 | 4-15 |
| Microcrystalline cellulose | 7.7 | 5-25 |
| Polyvinylpyrrolidone | 0.5 | 0.05-5 |
| Antioxidant | 0.2 | 0.1-5 |
| Sodium stearyl fumarate | 0.5 | 0.02-2 |
| **SECOND LAYER TABLET WEIGHT** | **16.6** | **10-20** |
| **TOTAL TABLET WEIGHT** | **97.6** | **95-99** |
| Coating materials | 2.4 | 0.1-5 |
| **FILM COATED TABLET WEIGHT** | **100** | **100** |

### Example 2:

| **Ingredients** | **% by weight** | **% by weight** |
|---|---|---|
| Metformin HCL | 60 | 40-80 |
| Sodium Carboxymethyl Cellulose (7 HF) | 3 | 1-7 |
| Hydroxypropyl methylcellulose (K100 LV) | 1.2 | 0.05-4 |
| Hydroxypropyl methylcellulose K100 MCR/ K100M/ CN10T | 16.4 | 10-35 |
| Sodium stearyl fumarate | 0.4 | 0.02-2 |
| **FIRST LAYER TABLET WEIGHT** | **81** | **75-85** |
| Sitagliptin phosphate monohydrate (eq to 100mg Sitagliptin) | 3.9 | 1-10 |
| Microcrystalline cellulose | 11.5 | 5-25 |
| Polyvinylpyrrolidone | 0.5 | 0.05-5 |
| Antioxidant | 0.2 | 0.1-5 |
| Sodium stearyl fumarate | 0.5 | 0.02-2 |
| **SECOND LAYER TABLET WEIGHT** | **16.6** | **10-20** |
| **TOTAL TABLET WEIGHT** | **97.6** | **95-99** |
| Coating materials | 2.4 | 0.1-5 |
| **FILM COATED TABLET WEIGHT** | **100** | **100** |

### A process for example 1-2;

### Metformin layer;

a) Granulating Metformin HCL, Carboxymethylcellulose sodium and Hydroxypropyl methylcellulose (K100LV) with pure water,
b) Drying the wet granules and sieving,
c) Adding Hydroxypropyl methylcellulose (K100 MCR/ K100M/ CN10T) and then mixing,
d) Adding Sodium stearyl fumarate and then mixing

### Sitagliptin layer;

e) Mixing Sitagliptin phosphate monohydrate and antioxidant,
f) Adding Polyvinyl Pyrrolidone and mixing,
g) Adding Microcrystalline cellulose and mixing,
h) Adding Sodium stearyl fumarate and mixing.

### Bilayer tablet compression;

i) Compressing layers as bilayer tablet to appropriate specifications defined.

### Film coating;

j) Coating the bilayer tablet with film coating.

### Example 3:

| **Ingredients** | **% by weight** | **% by weight** |
|---|---|---|
| Metformin HCL | 37 | 25-45 |
| Sodium Carboxymethyl Cellulose (7 HF) | 3.7 | 1-7 |
| Hydroxypropyl methylcellulose (K100 LV) | 0.7 | 0.05-4 |
| Hydroxypropyl methylcellulose K100 MCR/ K100M/ CN10T | 28 | 10-35 |
| Microcrystalline cellulose | 7.9 | 5-15 |
| Sodium stearyl fumarate | 0.4 | 0.02-2 |
| **FIRST LAYER TABLET WEIGHT** | **77.7** | **70-80** |
| Sitagliptin phosphate monohydrate (eq to 100mg Sitagliptin) | 4.7 | 1-10 |
| Microcrystalline cellulose | 14.3 | 5-25 |
| Polyvinylpyrrolidone | 0.6 | 0.05-5 |
| Antioxidant | 0.2 | 0.1-5 |
| Sodium stearyl fumarate | 0.6 | 0.02-2 |
| **SECOND LAYER TABLET WEIGHT** | **20.4** | **15-25** |
| **TOTAL TABLET WEIGHT** | **98.1** | **95-99** |
| Coating materials | 1.9 | 0.1-5 |
| **FILM COATED TABLET WEIGHT** | **100** | **100** |

## Claims

1. A bilayer tablet comprises;
• First layer comprising metformin or a pharmaceutically acceptable salt thereof and at least one cellulose derivatives binder and,
• Second layer comprising sitagliptin or a pharmaceutically acceptable salt thereof and at least one antioxidant.

2. The bilayer tablet according to claim 1, wherein at least one cellulose derivatives as binder are hydroxypropylcellulose, methyl cellulose, ethyl cellulose, hydroxypropyl methylcellulose, carboxymethylcellulose sodium or mixtures thereof.

3. The bilayer tablet according to claim 1 or 2, wherein cellulose derivatives binder is carboxymethylcellulose sodium and hydroxypropyl methylcellulose.

4. The bilayer tablet according to claim 1, wherein antioxidants are selected from the group comprising propyl gallate, meglumine, arginine, butylated hydroxy toluene, butylated hydroxy anisole, sodium metabisulfite, sodium bisulfite, alpha tocopherol, ascorbic acid, sodium ascorbate, ascorbyl palmitate, erythorbic acid, thioglycerols, thiogallic acid, cysteine, glutathione, cysteamine, dihydrolipoic acid, lipoic acid, thioredoxin, ethyl gallate, methyl gallate, lauryl gallate or mixtures thereof.

5. The bilayer tablet according to claim 4, wherein antioxidant is propyl gallate or meglumine or arginine or mixture thereof.

6. The bilayer tablet according to claim 1, wherein further comprises at least one pharmaceutically acceptable excipient is selected from the group comprising binders, matrix agents, fillers, lubricants, antioxidants, coating agents or mixtures thereof.

7. The bilayer tablet according to claim 6, wherein second layer comprises binder are selected from the group comprising polyvinylpyrrolidone (povidone), lactose anhydrous, pregelatinized starch, calcium carbonate, calcium phosphate dibasic, calcium phosphate tribasic, calcium sulphate, hydroxypropylcellulose, methyl cellulose, ethyl cellulose or mixtures thereof.

8. The bilayer tablet according to claim 7, wherein the second layer comprises a binder which is polyvinylpyrrolidone.

9. The bilayer tablet according to claim 6, wherein first layer comprises at least one the matrix agent which is selected from the group comprising hydroxypropylmethyl cellulose, ethyl cellulose, polymethylmethacrylate derivatives poloxamer polyvinyl alcohol, xanthan gum, sodium alginate, polymethacrylates, polyacrylamide, hydroxypropyl cellulose, polyvinyl acetate, cellulose acetate phthalate, ethylene vinyl acetate, methyl aminoethyl methacrylate, neutral methacrylic acid esters, polylactide, polylactide co-glycolide, diethyl aminoethyl methacrylate or mixtures thereof.

10. The bilayer tablet according to claim 9, wherein matrix agent is hydroxypropyl methyl cellulose (K100 MCR/ K100M/ CN10T).

11. The bilayer tablet according to claim 6, wherein fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

12. The bilayer tablet according to claim 11, wherein filler is microcrystalline cellulose.

13. The bilayer tablet according to claim 11 or 12, wherein filler is present at first layer or second layer or mixtures thereof.

14. The bilayer tablet according to claim 6, wherein lubricants are selected from the group comprising sodium stearyl fumarate, talc, magnesium stearate, calcium stearate, zinc stearate, magnesium lauryl sulfate, sodium oleate, polyethylene glycol, sodium lauryl sulphate or mixtures thereof.

15. The bilayer tablet according to claim 14, wherein lubricant is sodium stearyl fumarate.
